# EUROPEAN PATENT APPLICATION

(11) **EP 4 732 669 A1**
(43) Date of publication of application: **29.04.2026**
(21) Application number: 23946244.3
(22) Date of filing: 27.07.2023
(51) Int. Cl.: A01N 1/10, C12M 1/00, C12M 1/02, C12M 1/38

(54) **PERFUSION DEVICE AND PERFUSION METHOD FOR CRYOPRESERVATION OF BIOLOGICAL TISSUE**

(71) Applicant: Servare Biotechnology (Ningbo) Co., Ltd, Ningbo, Zhejiang 315514 (CN)
(72) Inventor: TAN, Yuansheng, Rancho Cucamonga, California 91701 (US); LIANG, Xinrong, Guangzhou, Guangdong 510375 (CN); YANG, Yi, Rancho Cucamonga, California 91701 (US); WU, Shanshan, Harbin, Heilongjiang 150515 (CN); CHEN, Xiaohong, Foshan, Guangdong 528247 (CN); ZHAO, Zijun, Guangzhou, Guangdong 510642 (CN)
(74) Representative: Huang, Liwei
(86) International application number: PCT/CN2023/109461
(87) International publication number: WO 2025/020157

(57) **Abstract**

The present disclosure relates to the technical field of biological sample preservation, and particularly, to an automatic perfusion apparatus and method for cryopreservation of biological tissues. The apparatus comprises a liquid supply module, a perfusion module, and a control module, wherein the liquid supply module is connected to the perfusion module through a liquid tubing, and the control module controls a liquid supply ratio and a liquid supply rate of the liquid supply module. The operation is simple. After the perfusion or recovery operation is completed on any biological sample, the operator only needs to replace the sample carrying plate or external carrying container with a new one and connect the sample carrying plate or external carrying container, after which a new round of operation can begin. Moreover, during the perfusion or recovery operation, the data processing module achieves automated control of the temperature source and the peristaltic pump according to the temperature-time working curve, the perfusion fluid flow rate-time working curve, and the perfusion fluid composition-time working curve. This ensures the continuity and consistency of the perfusion and recovery operations for each biological sample, eliminating the impact of human factors on the operation results.

## Description

### Technical Field

The present disclosure relates to the technical field of biological sample preservation, and particularly, to an automatic perfusion apparatus and method for cryopreservation of biological tissues.

### Background

Vitrification technology was initially successfully applied to the preservation of mouse embryos. It is a technique that converts a material into a glass-like amorphous body. In vitrified products, molecular kinetic energy is extremely low. Only small units, such as side groups or branches of polymers, are capable of movement, while molecular chains and chain segments are essentially immobilized in a frozen state. The mechanical properties exhibited under these conditions resemble those of glass, hence, this state is referred to as the glassy state, an intermediate state between the liquid and solid states. During this process, no ice crystals are formed, the molecules do not reorganize, and no significant molecular motion occurs. The physical properties of the glassy state make vitrification a method capable of reducing cryoinjury to cells and tissues and allowing long-term preservation of cells and tissues. Achieving vitrification requires a sufficiently rapid cooling rate. As the temperature decreases, the energy barrier becomes sufficiently high, and the viscosity rises to 1014 Pa·s. In this case, molecular conformations are effectively frozen, and diffusion-controlled material exchange and chemical reactions with the environment are kinetically hindered. Since most protein degradation pathways are driven by molecular mobility, the vitrified system exhibits excellent structural and chemical stability.

Vitrification-based preservation of biological tissues remains highly challenging, primarily due to the complex structures of organs and tissues and the diverse intercellular connections. Different structures require different cooling rates, and achieving uniform penetration of cryoprotectants is also highly difficult.

Under low-temperature conditions, the metabolic activity of living cells is significantly reduced, enabling long-term preservation of cells and tissues. However, unprotected direct freezing is lethal for most biological specimens, as the formation of intracellular and extracellular ice crystals alters the cellular environment and causes mechanical damage to the cells. Cellular damage under rapid cooling is primarily due to the formation of intracellular ice crystals, whereas cellular damage during slow cooling arises from mechanical interactions between intracellular and extracellular ice crystals. The addition of cryoprotective agents (CPAs) during freezing modulates water transport and inhibits ice crystal growth, thereby effectively reducing cellular damage and osmotic stress risk during freezing, enhancing cryopreservation outcomes, and making long-term preservation of cells, tissues, and organs feasible.

Cryoprotective agents (CPAs) are essential during the freezing process. CPAs can prevent cryoinjury through various mechanisms, such as minimizing solution effects, etc. CPAs are classified into permeating and non-permeating CPAs. Permeating CPAs function by penetrating the cells, balancing intracellular and extracellular osmotic pressure, avoiding excessive cell shrinkage, and reducing the formation of intracellular ice crystals. Common permeating cryoprotective agents comprise dimethyl sulfoxide (DMSO), ethylene glycol (EG), glycerol, and the like. Non-permeating CPAs do not cross the cell membrane but can induce cellular dehydration by modifying osmotic pressure, thereby reducing the formation of intracellular ice crystals. Non-permeating CPAs comprise two kinds of substances: oligosaccharides (most commonly sucrose and trehalose) and macromolecular compounds (such as albumin, polyvinylpyrrolidone, and the like).

During the vitrification process, the interaction between cryoprotectants and cryopreserved tissues and cells determines the formation of intracellular ice crystals. Consequently, the perfusion method of the cryoprotective solution within the tissue is also a critical factor influencing the success of vitrification-based preservation.

Currently, during vitrification-based preservation, the perfusion and infiltration of cryoprotective agents into tissues or cells, as well as the removal of cryoprotectants during tissue or cell thawing, are manually performed by operators. One limitation of manual operation is the need of considering the operator's workload. The concentration gradients of cryoprotective solutions are limited, that is, during tissue perfusion, it is not feasible to apply a large amount of cryoprotective solutions with different concentrations. As a result, cryopreserved tissue cells are exposed to cryoprotective solutions with different concentrations in a discontinuous manner, which can easily lead to excessive cell shrinkage. Moreover, the time intervals between successive exposures of cryopreserved tissues and cells to cryoprotective solutions at two different concentrations may further lead to improper cell shrinkage. Therefore, effectively enhancing the interaction between cryoprotective solutions and tissues and cells, enhancing the preservation and post-thaw viability of biological tissues, and optimizing the perfusion methods for tissues and cells are highly desirable objectives.

### Summary

The present disclosure provides a perfusion apparatus and method for cryopreservation of biological tissues.

### First aspect

The present disclosure provides an automatic perfusion apparatus for cryopreservation of biological tissues, wherein the apparatus comprises a liquid supply module, a perfusion module, and a control module, the liquid supply module is connected to the perfusion module through a liquid tubing, and the control module controls a liquid supply ratio and a liquid supply rate of the liquid supply module, as well as temperature regulation within the perfusion module.

### Liquid supply module:

The liquid supply module comprises reagent containers and peristaltic pumps disposed at openings of the reagent containers and connected to the reagent containers through tubings. The reagent containers hold liquids, comprising preservation solutions and cryoprotective solutions for biological tissues. Further, the preservation solution for the biological tissues may be the tissue fluid from the in vivo environment or a fluid analogous to the in vivo tissue fluid, and is configured to maintain the structural integrity and functional viability of the biological tissues.

The peristaltic pump transports the liquid from the reagent containers into the tubings where it is mixed, and subsequently delivers the mixed liquid into the perfusion module.

The number of the peristaltic pumps matches the number of the reagent containers, and the number of the reagent containers is determined according to the types of liquids to be supplied. When the liquids are classified as tissue fluid and cryoprotective solution, preferably two reagent containers are provided, one for tissue fluid and the other for cryoprotective solution. When the liquids are classified according to the individual components of the tissue fluid and cryoprotective solution, the number of reagent containers corresponds with the number of components.

After the peristaltic pumps pump liquids from the reagent containers, the liquids converge into a mixing tube for thorough mixing. The uniformly mixed liquid is then delivered through the tubings to the perfusion module.

Further, a mixing assembly may be disposed downstream of the mixing tube to further mix the mixed solution.

In a specific embodiment of the present disclosure, a branch tubing is disposed downstream of the mixing tube, serving as a detection tubing. The detection tubing is connected to an analytical apparatus to analyze whether the concentrations and ratios of the components in the mixed solution comply with the requirements of the control protocol. Perfusion module

The perfusion module comprises a temperature control plate and a flow channel plate pressed against the temperature control plate to form a sealed chamber, wherein a sample carrying plate is disposed within the sealed chamber, the sample carrying plate is provided with a plurality of sample carrying spaces for accommodating biological samples, and adjacent sample carrying spaces are connected by means of flow channels. After the mixed solution enters the sample carrying spaces, it permeates into the biological sample, displacing the tissue fluid and/or cryoprotective solution in the biological sample according to a concentration gradient. The displaced liquids are discharged from the perfusion module through the flow channels and are collected.

Further, the sample carrying plate and the flow channel plate in the perfusion module may be fabricated in different structures according to different biological samples.

In one embodiment of the present disclosure, the biological samples are tissue sections, and the sample carrying plate is a geometrically shaped flat plate, preferably provided with spaces for accommodating the tissue sections. In another embodiment of the present disclosure, the biological samples are tissue blocks, organoids, or organs, and the sample carrying plate is a carrying container. The carrying container is provided with liquid flow channels to allow the inflow and outflow of tissue fluid or cryoprotective solution. In yet another embodiment of the present disclosure, the biological samples are cellular or viral tissues, and the sample carrying plate is a tube-like container. The container is further provided with liquid flow channels to allow the inflow and outflow of tissue fluid or cryoprotective solution.

Further, the biological sample of the present disclosure is selected from cells, viruses, organoids, tissues, oocytes, sperm, embryos, and various physiological or pathological organ sections, such as sections of heart, liver, lung, ovary, kidney, cartilage, blood vessels, valves, or cornea.

Further, the sample carrying plate of the present disclosure may be disposed in the perfusion module in a plug-in configuration.

Further, the sample carrying container of the present disclosure may be externally connected to the perfusion module.

### Control module:

The control module comprises a temperature control assembly, a liquid-supply control assembly, and a display assembly.

The temperature control assembly comprises a cold-source contact surface disposed to abut the bottom of the temperature control plate, and a cooling component.

The cold-source contact surface interfaces with an external cold source and exchanges heat with the temperature control plate, thereby controlling the temperature of the perfusion module. Preferably, the temperature control assembly further comprises a temperature sensor for detecting the temperature.

The cooling component comprises a cooling platform, which comprises a plurality of supporting bases connected to the cold source. The supporting bases exchange heat with the cold source, and tops of the supporting bases are coplanar to form a cooling plane. A side of the cooling plane that contacts the temperature control plate serves as the cold-source contact surface. The perfusion module is placed on the cooling plane and is in contact with the supporting bases, thereby ensuring efficient heat exchange between the perfusion module and the cooling module.

The liquid-supply control assembly regulates the liquid supply rate and liquid supply composition by controlling the working state of the peristaltic pumps, specifically by adjusting the peristaltic speed of each peristaltic pump.

Different biological samples require varying perfusion flow rates, temperatures, and liquid supply compositions. For each biological sample, a perfusion fluid flow rate-time working curve and a perfusion fluid liquid supply composition-time working curve can be established. By programming the working curves into the system for different biological samples, automated perfusion of the perfusion fluid can be realized.

Further, the liquid-supply control assembly further comprises a flow-rate detector disposed downstream of the mixing tube for monitoring the liquid supply flow rate, and a tissue fluid concentration detector and/or a cryoprotective solution concentration detector for monitoring the concentration of the mixed solution.

The display assembly comprises an integrated display screen with data-processing functionality, which controls the temperature of the temperature control assembly and provides real-time feedback of the temperature detected by the temperature sensor. The liquid-supply control assembly provides periodic data on the operation of the peristaltic pumps, enabling the peristaltic pumps to run automatically during perfusion. Data detected by the flow-rate detector and the tissue fluid concentration detector or cryoprotective solution concentration detector are fed back to the display screen, allowing operators to monitor and adjust the liquid supply flow rate and the liquid concentration ratios at relevant time points. The display assembly, liquid-supply control assembly, and temperature-control assembly are connected to an external power source.

Further, the display assembly is preloaded with corresponding control protocols for various biological samples. The control protocols comprise working curves and temperature-selection protocols specific to each biological sample. The working curves comprise the perfusion fluid flow rate-time working curve and the perfusion fluid liquid supply composition-time working curve.

The control protocols control the real-time working states of the cooling module and the liquid supply module. In an embodiment of the present disclosure, the display screen further comprises a function-selection key for the biological samples, with different biological samples corresponding to different control protocols.

### Second aspect

The present disclosure provides an automatic perfusion method for cryopreservation of biological tissues, the method comprising the following steps:
In S01, a corresponding sample carrying plate is selected according to a biological sample to be perfused, and the biological sample is placed in the sample carrying plate.
In S02, the biological sample is selected via a display assembly so that the biological sample corresponds to a suitable perfusion control protocol, wherein the perfusion control protocol involves loading a working curve adapted to the corresponding biological sample and types of reagent solutions into a data processing system to form the perfusion control protocol.

On the basis of the perfusion control protocol, the data processing system controls working states of a temperature source and peristaltic pumps, adjusts output power of the temperature source so that real-time temperature of a sample carrying plate follows a temperature-time working curve, and adjusts the peristaltic speed of each peristaltic pump to regulate the feed rate of the reagents so that the feed rate follow the corresponding perfusion fluid working curve.

In S03, the peristaltic pumps aspirate under the control of a perfusion protocol, liquids from the corresponding reagent containers before merging into a mixing tube. After uniform mixing in the mixing tube, the liquids enter a perfusion module through flow channels, flow, and diffuse into sample carrying spaces or liquid channels of the sample carrying plate to perfuse the biological sample.

Further, a mixing assembly is disposed downstream of the mixing tube to further mix the liquid, ensuring thorough mixing of the liquid.

In S04, excess mixed liquid in the sample carrying spaces or carrying channels, or liquid exuded from the biological sample flow out through outlets of the flow channels and is collected.

In S05, upon completion of the perfusion protocol, the biological sample alone or the sample carrying plate carrying the biological sample is removed, is placed in a cooling apparatus and is then placed in liquid nitrogen or a low-temperature storage device.

Further, the temperature-time working curve is generated by taking the duration of various stages of the perfusion, infiltration, and/or recovery process of the biological sample as the axis and sequentially connecting the temperature values or ranges required in each stage of the biological sample.

When the temperature-time working curve is loaded into a data processing module, the data processing module controls the output power of the temperature-control module at each stage on the basis of the temperature values of the temperature-time working curve, so that the temperature of the biological sample conforms to the settings defined by the temperature-time working curve. Further, by controlling the working time of the temperature control module at each stage, the temperature of the space containing the biological sample is controlled, so that the temperature of the biological sample conforms to the settings defined by the temperature-time working curve.

Preferably, temperature control is achieved by multi-core semiconductor cooling, where the cooling effect is realized via the Peltier effect, caused by passing current through semiconductor materials. The temperature-time working curve is implemented using a PID algorithm.

The perfusion fluid flow rate-time working curve and the perfusion fluid composition-time working curve are generated by taking the duration of various stages of the perfusion, infiltration, and/or recovery process of the biological sample as the axis and sequentially connecting the corresponding feed rate and feed speed of the reagent solution delivered by the peristaltic pump at each stage. Alternatively, the perfusion fluid flow rate-time working curve and the perfusion fluid composition-time working curve of the reagent solution can be adjusted according to feedback information from the sensor.

According to the type of the biological sample, the type of reagent solution, and the requirements for perfusion, infiltration, and/or recovery operations, each reagent solution has its own perfusion fluid flow rate-time working curve and perfusion fluid composition-time working curve. Correspondingly, the peristaltic pump delivering the reagent solution also has its own perfusion fluid flow rate-time working curve and perfusion fluid composition-time working curve.

Preferably, when the perfusion fluid flow rate-time working curve and the perfusion fluid composition-time working curve of each peristaltic pump are loaded into the data processing module, the corresponding feed rate and feed speed of the reagent solution at each stage are directly input into the data processing module. On the basis of the feed rate and feed speed from the perfusion fluid flow rate-time working curve and the perfusion fluid composition-time working curve, the data processing module controls the operating frequency or operating speed of the peristaltic pump to achieve precise control of the feed rate of the reagent solution, so that each reagent solution conforms to the settings defined by the perfusion fluid flow rate-time working curve and perfusion fluid composition-time working curve.

Preferably, the feed rate corresponding to the perfusion fluid flow rate-time working curve is converted into operating frequencies or operating speeds of the peristaltic pumps, and the data processing module reads the operating frequency or the operating speed of each peristaltic pump and adjusts the feed rate of each peristaltic pump to realize precise control of the feed rate of the reagent solutions, so that each reagent solution conforms to the settings defined by the perfusion fluid flow rate-time working curve and perfusion fluid composition-time working curve.

Preferably, during various stages of the perfusion, infiltration, and/or recovery process of the biological sample, the temperature-time working curve, the perfusion fluid flow rate-time working curve, and the perfusion fluid composition-time working curve are divided into curves for each time interval on the basis of time intervals corresponding to each stage. The curves for each time interval are set using the same function, ensuring that the working curve forms a smooth, continuous curve, or is set using different functions to meet the temperature or temperature change requirements for the biological sample during each stage of perfusion, infiltration, and/or recovery process, as well as the feed rate and feed speed requirements for each reagent solution during each stage of perfusion, infiltration, and/or recovery process.

Preferably, the data processing module receives a signal indicating the real-time temperature of the sample carrying module and determines whether the real-time temperature of the sample carrying module corresponds to a temperature value at the current time point on the temperature-time working curve; if so, the data processing module maintains output power of a temperature-control module according to the temperature-time working curve; if not, the data processing module adjusts output power of a cooling module of the temperature-control module to compensate for temperature deviation of the sample carrying module so that the real-time temperature of the sample carrying module reaches the temperature value at the current time point on the temperature-time working curve; and thereafter the temperature-control module is restored to output power corresponding to the temperature-time working curve.

Preferably, according to the difference adjusted by the data processing module, the delivery parameters are adjusted so that the feed rate and feed speed conform to the settings defined by the perfusion fluid flow rate-time working curve and the perfusion fluid composition-time working curve.

With the use of this biological sample perfusion apparatus, the operator can perform large-scale, rapid perfusion or recovery operations on the same biological sample. After the perfusion or recovery operation is completed on any biological sample, the operator only needs to replace the sample carrying plate or external carrying container with a new one and connect the sample carrying plate or external carrying container, after which a new round of operation can begin. Moreover, during the perfusion or recovery operation, the data processing module achieves automated control of the temperature source and the peristaltic pump according to the temperature-time working curve, the perfusion fluid flow rate-time working curve, and the perfusion fluid composition-time working curve. This ensures the continuity and consistency of the perfusion and recovery operations for each biological sample, eliminating the impact of human factors on the operation results.

The biological sample perfusion method of the present disclosure is suitable for large-scale low-temperature vitrification cryopreservation of biological samples. On the basis of the specifications of the low-temperature vitrification cryopreservation container, the specifications of the sample carrying plate or the external carrying container are designed so that the sample carrying plate and the external carrying container can serve as carriers for loading biological sample slices during perfusion operations, vitrification cryopreservation operations, and biological sample recovery operations. As carriers for carrying biological samples, the sample carrying plate and the external carrying container can be directly transferred to a low-temperature storage unit after the perfusion is completed. The sample carrying plate and the external carrying container undergo low-temperature vitrification cryopreservation in the liquid nitrogen-cooled low-temperature storage unit. During large-scale low-temperature vitrification cryopreservation of biological samples, the operator only needs to repeatedly replace the sample carrying plate or the external carrying container to complete batch operations, achieving modular perfusion operations of biological samples and greatly simplifying the complexity of batch operations.

Compared to the prior art, the automatic perfusion apparatus for cryopreservation of biological tissues according to the present disclosure has the following technical advantages:
1. The biological sample perfusion apparatus of the present disclosure has a wide application range. The biological sample perfusion apparatus of the present disclosure may be suitable for different types of biological samples and tissues. When operating on different types of biological samples, only the corresponding sample carrying plate or flow channel plate needs to be replaced, eliminating the need of replacing the entire apparatus. This makes it suitable for large-scale operations of biological samples and tissues.
2. The biological sample perfusion apparatus of the present disclosure continuously distributes cryoprotective solution or tissue fluid to the biological sample by means of the perfusion unit module, avoiding the negative effects of discontinuous delivery, which can cause cell shrinkage.
3. The biological sample perfusion apparatus of the present disclosure achieves automated control of biological sample and tissue perfusion, infiltration, and recovery operations via the data processing module. There is no need for manual preparation of different concentrations of cryoprotective solution or tissue fluid, eliminating operational errors caused by human factors and enhancing the stability of biological samples and tissue perfusion, infiltration, and recovery operations.
4. The biological sample perfusion apparatus of the present disclosure monitors and adjusts the working state of the liquid supply module in real time, ensuring more precise delivery of cryoprotective solution or tissue fluid compared to manual mixing. This allows for seamless transitions between low and high concentrations, or vice versa, ensuring thorough and safe replacement of intracellular and extracellular water. This process minimizes the formation of ice crystals during cooling, thereby preserving the sample's viability.
5. The biological sample perfusion apparatus of the present disclosure monitors and adjusts the working load of the cooling unit in real time, enabling precise temperature control throughout the entire perfusion and infiltration process of the biological sample. This maximizes the retention of the sample's viability.

### Brief Description of the Drawings

FIG. 1 is a schematic diagram of an overall structure of an automatic perfusion apparatus for cryopreservation of biological tissues;
FIG. 2 is a schematic structural diagram of a semiconductor cooling module of an automatic perfusion apparatus for cryopreservation of biological tissues;
FIG. 3 is a schematic structural diagram of a perfusion unit module of an automatic perfusion apparatus for cryopreservation of biological tissues;
FIG. 4 is a schematic structural diagram of a flow channel surface of an automatic perfusion apparatus for cryopreservation of biological tissues;
FIG. 5 is a schematic diagram of an internal cross-sectional structure of a flow channel plate of an automatic perfusion apparatus for cryopreservation of biological tissues;
FIG. 6 is a schematic structural diagram of a sealing surface of an automatic perfusion apparatus for cryopreservation of biological tissues;
FIG. 7 is a schematic structural diagram of a sample carrying plate of an automatic perfusion apparatus for cryopreservation of biological tissues;
FIG. 8 is a schematic structural diagram of a temperature control plate of an automatic perfusion apparatus for cryopreservation of biological tissues;
FIG. 9 is a schematic structural diagram of a mixing assembly of an automatic perfusion apparatus for cryopreservation of biological tissues;
FIG. 10 is a schematic structural diagram of an automatic perfusion method for cryopreservation of biological tissues; and

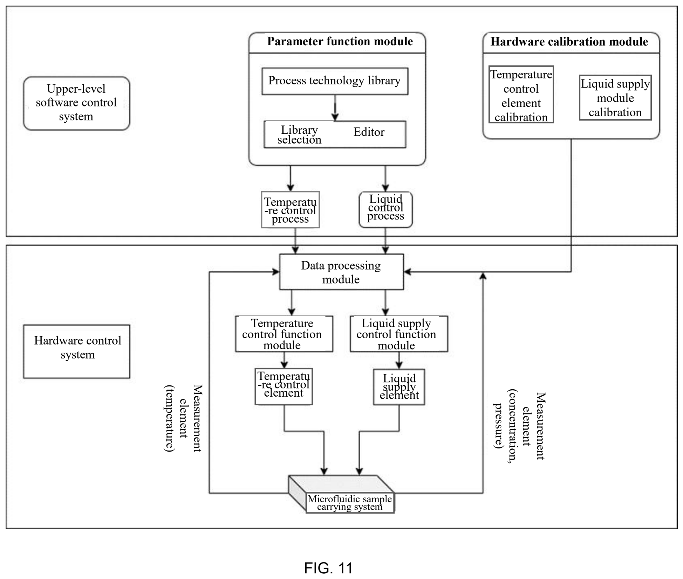

FIG. 11 is a schematic flowchart of an automatic perfusion method for cryopreservation of biological tissues.

### Reference numerals:

1. Housing; 2. Semiconductor cooling module; 3. Peristaltic pump; 4. Reagent container; 5. Mixing tube; 6. Supporting base; 7. Cooling plane; 8. Perfusion unit module; 9. Temperature control plate; 10. Sample carrying plate; 11. Flow channel plate;12. Sealing plate; 13. Cold-source contact surface; 14. Sample carrying surface; 15. Primary positioning edge; 16. Positioning space; 17. Secondary positioning edge; 18. Secondary positioning space; 19. Drainage port; 20. Recovery port; 21. Sample carrying space; 22. Infiltration port; 23. Flow channel surface; 24. Sealing surface; 25. Flow channel; 26. Positioning hole; 27. Sealing groove; 28. Through hole; 29. Gasket; 30. Flow channel opening; 31. First perfusion channel; 32. Second perfusion channel; 33. Third perfusion channel; 34. Inlet port; 35. First perfusion passage; 36. Second perfusion passage; 37. Inlet channel; 38. First channel; 39. Second channel; 40. Third channel; 41. Mixing assembly; 42. Mixing member; 43. Mixing sheet.

### Detailed Description of the Embodiments

To further elaborate on the technical means and effects adopted by the present disclosure to achieve the intended objectives, the following detailed description of the implementations, structure, features, and effects according to the present disclosure is provided, with reference to the drawings and preferred embodiments.

### Embodiment 1: Automatic perfusion apparatus for cryopreservation of biological tissues

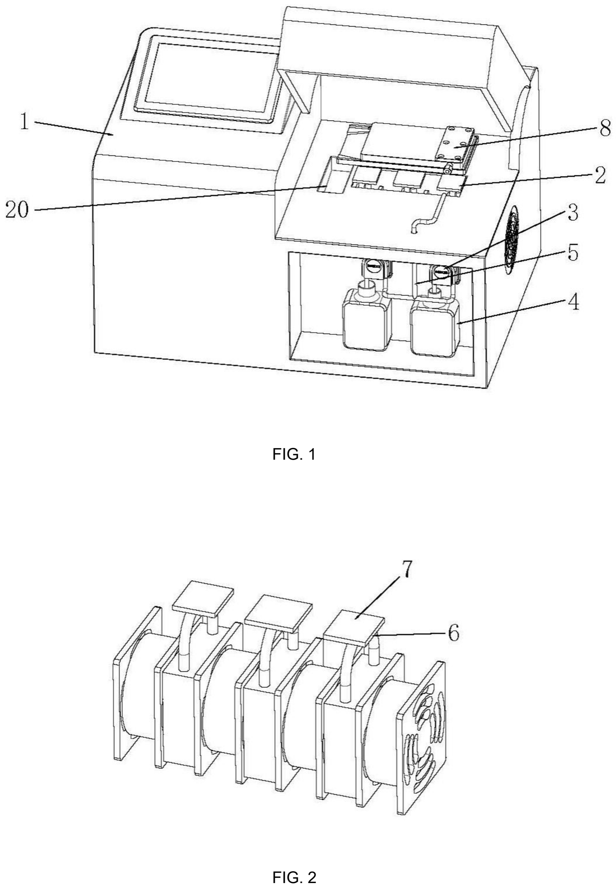

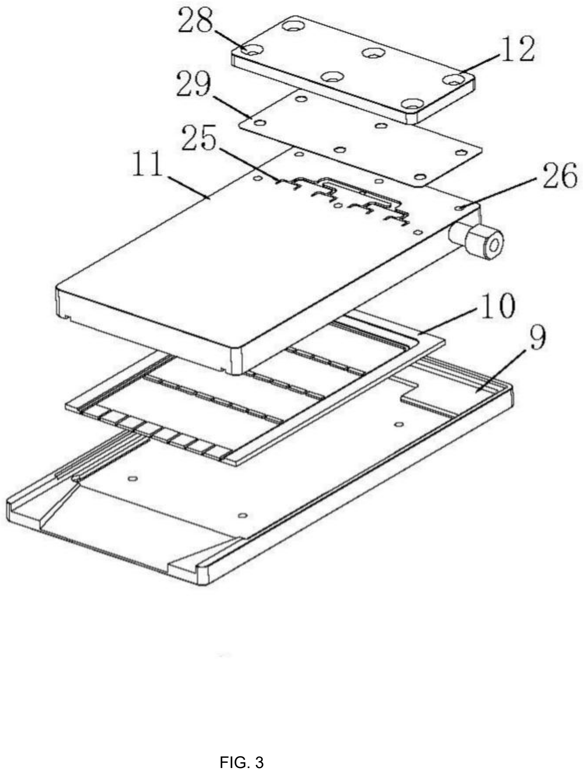

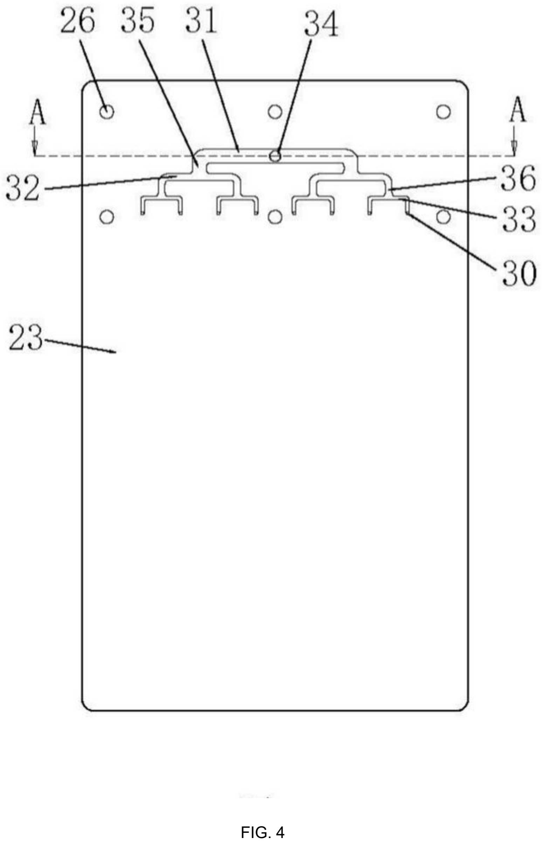

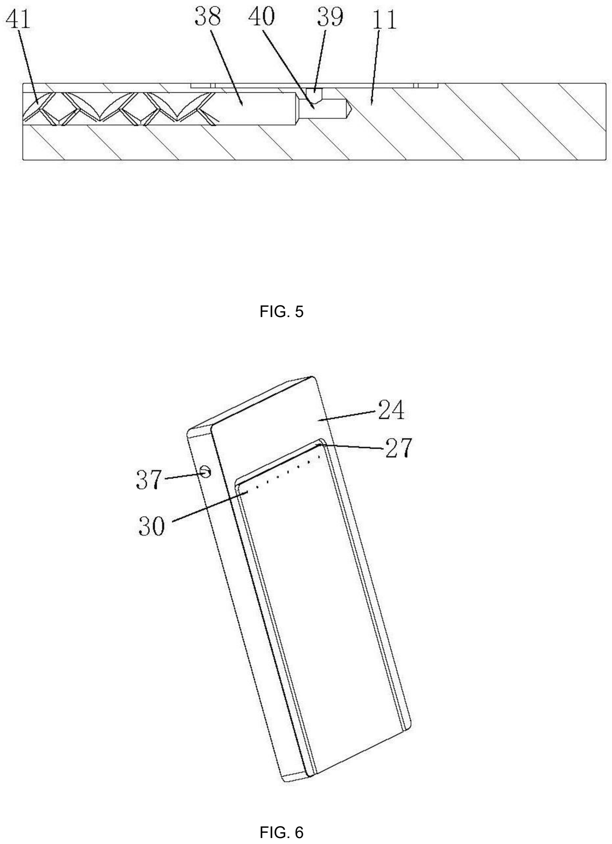

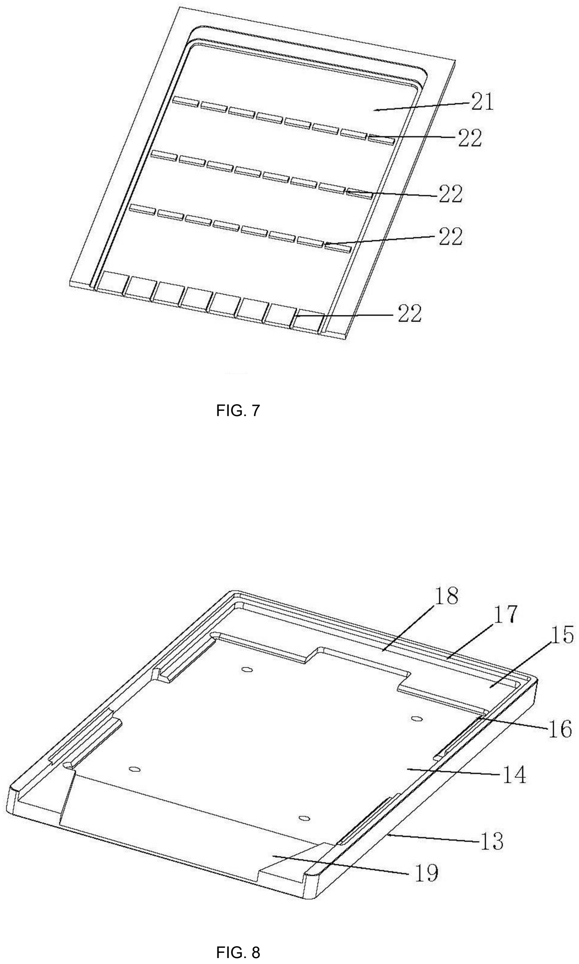

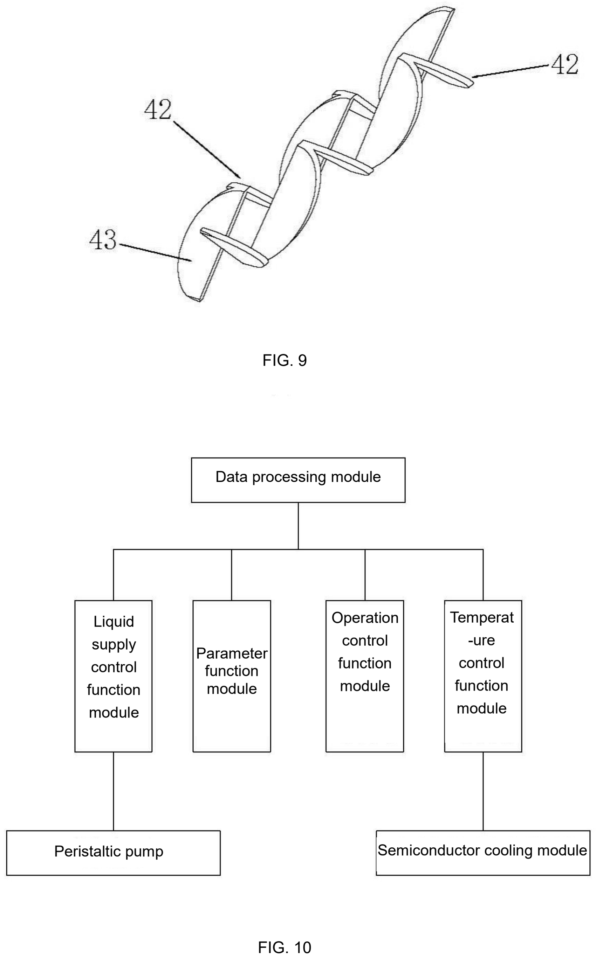

### As shown in FIGS. 1 to 9

An automatic perfusion apparatus for cryopreservation of biological tissues comprises an apparatus body, wherein the apparatus body comprises a housing 1, and within the housing 1 are disposed a temperature control assembly 2 and a liquid supply module. In this embodiment, the liquid supply module comprises two peristaltic pumps 3 acting as liquid feeding pumps and two reagent containers 4. Inlet ports of the peristaltic pumps 3 are in separate communication with the corresponding reagent container 4, and outlet ports of the peristaltic pumps 3 are interconnected via a three-way tubing. At the junction of the outlet ports, i.e., downstream of the mixing tube 5, the three-way tubing is connected to a detection tubing, which is in turn connected to an analytical apparatus.

The temperature control assembly 2 is provided with a cooling platform featuring an open design, serving as the interfacing space for the cold source supply. The cooling platform comprises three supporting bases 6 connected to the cold source. The support bases 6 are made of a metal material, which allows for maintaining high heat exchange efficiency with the cold source. The tops of the supporting bases 6 are coplanar to form a cooling plane 7, and the perfusion module 8, separated from the apparatus body, is placed on the cooling plane 7 and connected to the supporting bases 6 in a surface-contact manner. The housing is further provided with a hinged flip cover, so that the perfusion unit module 8 can be covered inside, achieving the purpose of protection. According to different cooling methods, the temperature control assembly 2 can also be replaced with a water tank, utilizing a temperature control method that combines an ice-water bath and heating rods. Temperature control of the cooling module is achieved through the working state of an externally connected compressor. In this case, the inside of the water tank serves as the interfacing space for placing the perfusion module.

The perfusion unit module 8 comprises a temperature control plate 9, a sample carrying plate 10, a flow channel plate 11, and a sealing plate 12.

The temperature control plate 9 is made of a metal material with a plate-like structure. A cold-source contact surface 13 and a sample carrying surface 14 are arranged oppositely on the temperature control plate 9. The cold-source contact surface 13 is a continuous plane. The sample carrying surface 14 is provided with a primary positioning edge 15 that protrudes from the sample carrying surface 14. The primary positioning edge 15 is arranged in a surrounding pattern on the sample carrying surface 14, forming a positioning space 16. A secondary positioning edge 17, which protrudes from the primary positioning edge 15, is further disposed on the primary positioning edge 15. The secondary positioning edge 17 is arranged in a surrounding pattern on the primary positioning edge 15, forming a secondary positioning space 18. The sample carrying surface 14 of the temperature control plate 9 is provided with a drainage port 19 in communication with the outside. The drainage port 19 has an inclined surface relative to the sample carrying surface 14 to allow fluid to flow out of the drainage port 19. The housing 1 is further provided with a recovery port 20, and the recovery port 20 is located below the drainage port 19 and in communication with the drainage port 19. A tubing, connected to a recovery container, is disposed at the bottom of the recovery port 20.

The sample carrying plate 10 is made of a metal material with a plate-like structure and is mounted in the positioning space 16, with the top of the sample carrying plate 10 flush with the top of the primary positioning edge 15. The bottom of the sample carrying plate 10 is a continuous plane and is in contact with the sample carrying surface 14. The sample carrying surface 14 achieves heat exchange through surface contact. The sample carrying plate 10 is provided with four sample carrying spaces 21 for carrying biological samples, and the four sample carrying spaces 21 are arranged sequentially. The first three sample carrying spaces 21 are provided with a plurality of infiltration ports 22 in communication with adjacent sample carrying spaces 21, and the last sample carrying space 21 is provided with a plurality of infiltration ports 22 in communication with the drainage port 19.

The flow channel plate 11 has a plate-like structure and is mounted in the secondary positioning space 18. The flow channel plate 11 is provided with a flow channel surface 23 and a sealing surface 24 arranged oppositely to each other. The flow channel 25 is disposed on the flow channel surface 23, with the top opening of the flow channel 25 communicating with the outside. The flow channel surface 23 is further provided with a plurality of positioning holes 26, and the positioning holes 26 are arranged around the flow channel 25. The sealing surface 24 is a continuous plane, and the sealing surface 24 is provided with a sealing groove 27 for filling a sealing strip. When the sealing surface 24 is pressed against the sample carrying plate 10, the sealing groove 27 surrounds the outside of the sample carrying space 21.

The sealing plate 12 has a plate-like structure, and through holes 28 are disposed in the sealing plate 12 at positions corresponding to the positioning holes 26. The operator can fix the sealing plate 12 onto the flow channel surface 23 using screws, covering the flow channel 25 on the flow channel plate 11 to avoid fluid from overflowing during the perfusion operation. A gasket 29 is disposed between the sealing plate 12 and the flow channel plate 11 to ensure the sealing of the flow channel 25 by the sealing plate 12.

The flow channel plate 11 is pressed against the sample carrying plate 10, and the sealing strip adheres to the sample carrying plate 10, ensuring that each sample carrying space 21 forms a sealed space. The flow channel plate 11 is provided with eight flow channel openings 30, and the eight flow channel openings 30 are evenly arranged and in communication with the first sample carrying space 21. The flow channel plate 11 is provided with flow channels 25 in communication with each flow channel opening 30. In particular, the flow channels 25 comprise a first perfusion channel 31, a second perfusion channel 32, and a third perfusion channel 33.

An inlet port 34 is disposed in the middle of the first perfusion channel 31, and channel outlets are disposed at two ends of the first perfusion channel 31, respectively. The channel outlets are in communication with the middle of the second perfusion channel 32, forming a first perfusion passage 35.

Channel outlets are disposed at two ends of the second perfusion channel 32. The channel outlets are in communication with the middle of the third perfusion channel 33, forming a second perfusion passage 36.

Channel outlets are disposed at two ends of the third perfusion channel 33. The channel outlets are in communication with the flow channel openings 30, and the third perfusion channel 33 is not interconnected. The channel inner diameters of the first perfusion channel 31, the second perfusion channel 32, and the third perfusion channel 33 decrease sequentially.

In this embodiment, the flow channel plate 11 is provided with an inlet channel 37. The inlet channel 37 comprises the first channel 38, the second channel 39, and the third channel 40 that are interconnected. The channel inner diameters of the first channel 38, the third channel 40, and the second channel 39 decrease sequentially. The second channel 39 is arranged vertically to the flow channel surface 23 and in communication with the third channel 40. The first channel 38 is in communication with the inlet port 34, and a mixing assembly 41 is inserted into the first channel 38. The mixing assembly 41 comprises two mixing members 42 arranged in parallel. Each mixing member 42 comprises a plurality of mixing sheets 43 with a plate-like structure. The adjacent mixing sheets 43 within the same mixing member 42 are connected end to end, and the corresponding mixing sheets 43 in the two mixing members 42 are cross-connected.

In this embodiment, the housing 1 is provided with a data processing module therein. The data processing module is provided with a liquid supply control function module, a temperature control function module, a parameter adjustment function module, and an operation control function module therein.

In this embodiment, the housing 1 is provided with a control module therein, the control module comprising
a temperature sensor for detecting the temperature of the temperature control assembly 2.

In an example of the biological sample perfusion operation, the working process of the biological sample perfusion apparatus in this embodiment is as follows:
First, according to the type and quantity of the biological samples to be processed, the control protocol preset in the parameter adjustment function module is read. Then, on the basis of the reagent solution types and amount shown in the control protocol, the appropriate reagent solutions are selected and added to the reagent containers 4.

The sample carrying plate 10 loaded with the biological samples to be processed is placed on the sample carrying surface 14. The flow channel plate 11 is pressed onto the sample carrying plate 10, forming the perfusion unit module 8. The entire perfusion unit module 8 is then placed on the cooling platform of the semiconductor cooling module 2, and the cold-source contact surface 13 is connected to the cooling plane 7 in a surface-contact manner. The housing 1 is provided with a pressing apparatus, which presses the flow channel plate 11 onto the sample carrying plate 10, ensuring the sealing between the flow channel plate 11 and the sample carrying plate 10.

The parameter function module is used to acquire the perfusion control protocol corresponding to the biological sample, such as the reagent solution ratio, the working curves of each peristaltic pump 3, and the temperature-time working curve of the semiconductor cooling module 2, etc. The reagent solutions required for the current perfusion and infiltration operation are placed onto the corresponding solution holders. According to the perfusion control protocol, each reagent solution is connected to the inlet of the corresponding peristaltic pump 3, and the three-way tubing is connected to the inlet channel 37 of the flow channel plate 11.

Upon activation of the display assembly, the operation control function module reads the control protocol selected by the parameter adjustment function module and outputs control signals to the liquid supply control function module and the temperature control function module on the basis of the selected control protocol. The liquid supply control function module, on the basis of the control signals, controls the feed frequency and feed time of each peristaltic pump 3, allowing the reagent solution to enter the mixing junction, i.e., the mixing tube, for preliminary mixing. During this process, the temperature control function module monitors the real-time temperature of the perfusion unit module 8 to ensure that the real-time temperature of the perfusion unit module 8 matches the current time-temperature value of the temperature-time working curve.

During the cooling process of the sample carrying plate 10, the temperature probes arranged on the temperature control plate 9 monitor the real-time temperature of the temperature control plate 9 and transmit the temperature signal to the data processing module. The operation control function module selects, according to the current time-temperature value of the temperature-time working curve and the actual temperature of the sample carrying plate 10 at the current time, the output power of the semiconductor cooling module 2 and transmits this output power to the temperature control function module. The temperature control function module then controls the operation of the semiconductor cooling module 2, compensating for the temperature deviation of the sample carrying plate 10 to quickly bring the actual temperature of the sample carrying plate 10 to match the current time-temperature value of the temperature-time working curve. According to the changes in the temperature-time working curve, the data processing module continuously adjusts the control output according to the stage of the biological sample, dynamically modifying the working state of the semiconductor cooling module 2 to ensure that the temperature of the biological sample conforms to the settings defined by the temperature-time working curve.

Subsequently, the operation control function module dynamically adjusts the set value of the output power of the semiconductor cooling module 2 according to the rate of temperature change over the time period T of the temperature-time working curve. This set value is then transmitted in real-time to the temperature control function module, which controls the operation of the semiconductor cooling module 2 to ensure that the actual temperature of the sample carrying plate 10 decreases according to the set temperature-time working curve, reaching the set temperature within the specified time period T. Then, the operation control function module transmits a signal to the temperature control function module on the basis of the temperature-time working curve to maintain the output power of the semiconductor cooling module 2, ensuring that the temperature of the sample carrying plate 10 remains at the set temperature.

After the semiconductor cooling module 2 has been running for a period of time and the sample carrying plate 10 has reached the appropriate temperature, the operation control function module transmits a control signal to the liquid supply control function module according to the working curves of each peristaltic pump 3.

The liquid supply control function module controls, on the basis of the control signal, the feed speed, feed composition, and feed time of each peristaltic pump 3. At the starting time point set by the working curve, the activation of the peristaltic pump is controlled. Subsequently, according to the feed rate, feed composition, and the single feed capacity of the peristaltic pump 3 set for the current time by the working curve, pulse signals are transmitted to control the operating frequency of the peristaltic pump 3. This ensures that the feed working curve of the peristaltic pump 3 matches the feed rate set for the current time. The reagent solution flows from the outlet of the peristaltic pump 3 into the delivery tubing, where the reagent solution undergoes preliminary mixing at the mixing junction, i.e., the mixing tube, and then enters the first channel 38 through the inlet port 34. After thorough mixing within the first channel 38 via the mixing assembly 41, the reagent solution sequentially enters the third channel 40 and the second channel 39, slowly flowing into the flow channel 25 of the flow channel plate 11. According to the changes in each working curve, the data processing module continuously adjusts the control output according to the stage of the biological sample, dynamically modifying the working state of each peristaltic pump 3 to ensure that the feed parameters of each reagent solution conform to the settings defined by the working curve.

The cryoprotective solution obtained from the mixing of the reagent solutions flows into the flow channel 25, then sequentially diverts through the first perfusion channel 31, the second perfusion channel 32, and the third perfusion channel 33, flowing in equal amounts into the flow channel opening 30 and entering the sample carrying plate 10. A heat exchange space that is in communication with the sample carrying space 21 can be disposed upstream of the sample carrying plate 10 in the sample carrying space. After entering the sample carrying plate 10, the cryoprotective solution first flows through the heat exchange space, exchanging heat with the sample carrying plate 10 until the temperature of the sample carrying plate 10 becomes isothermal. The cryoprotective solution then flows into the first sample carrying space 21 of the sample carrying plate 10 and performs synchronized perfusion of the biological sample within the same sample carrying space 21. The first perfusion channel 31, the second perfusion channel 32, and the third perfusion channel 33 perform multi-level diversion, which greatly ensures that the cryoprotective solution is evenly distributed into the sample carrying space 21, enabling synchronized perfusion of the biological sample within the sample carrying space 21. Through the infiltration ports 22 of the sample carrying spaces 21, the cryoprotective solution uniformly soaks the biological samples within the sample carrying spaces 21. Subsequently, the displaced cell tissue fluid and the remaining cryoprotective solution flow out from the infiltration port 22 of the last sample carrying space 21 to the drainage port 19, then are discharged to the recovery port 20, and flow back to the recovery container via the recovery port 20.

After completing the perfusion and infiltration operation for the first batch of biological samples, the operator removes the sample carrying plate 10 and places the sample carrying plate 10 directly into the ultra-low temperature storage container for storage. Subsequently, the operator places a new sample carrying plate 10 loaded with the same type of biological samples into the temperature control plate 9 and repeats the perfusion and infiltration operation process, thereby completing the large-scale perfusion and infiltration operation for the same type of biological samples.

In addition, when processing biological tissues of very small sizes, the apparatus can also add a carrying container connected to the perfusion unit module, and the biological tissue is loaded by means of the carrying channel disposed inside the carrying container. When the carrying container is connected to the sample carrying module, the carrying channel is in communication with the sample carrying space 21. In this case, unlike the carrying function of the sample carrying space 21 in carrying the biological samples, the sample carrying space 21 serves to divert and cool the mixed solution.

The above is only a preferred embodiment of the present disclosure and does not impose any form of limitation on the present disclosure. Although the present disclosure has been disclosed in the preferred embodiment, it is not intended to limit the present disclosure. Any person skilled in the art, without departing from the scope of the technical solution of the present disclosure, may make some modifications or adjustments using the technical content disclosed above, which may result in equivalent variations or equivalent embodiments. The modifications, equivalent variations, and modifications made to the embodiments above according to the technical essence of the present disclosure, as long as they do not depart from the content of the technical solution of the present disclosure, are still within the scope of the technical solution of the present disclosure.

## Claims

1. An automatic perfusion apparatus for cryopreservation of biological tissues, comprising a liquid supply module, a perfusion module, and a control module, wherein the liquid supply module is connected to the perfusion module through a liquid tubing, and the control module controls a liquid supply ratio and a liquid supply rate of the liquid supply module.

2. The automatic perfusion apparatus for cryopreservation of biological tissues according to claim 1, wherein the liquid supply module comprises reagent containers and peristaltic pumps disposed at openings of the reagent containers and connected to the reagent containers through tubings, the number of the peristaltic pumps matches the number of the reagent containers, and the number of the reagent containers is determined according to types of liquids to be supplied.

3. The automatic perfusion apparatus for cryopreservation of biological tissues according to claim 2, wherein a mixing assembly is disposed downstream of a mixing tube.

4. The automatic perfusion apparatus for cryopreservation of biological tissues according to claim 1, wherein the perfusion module comprises a temperature control plate and a flow channel plate pressed against the temperature control plate to form a sealed chamber, a sample carrying plate is disposed within the sealed chamber, the sample carrying plate is provided with a plurality of sample carrying spaces for accommodating biological samples, and adjacent sample carrying spaces are connected through flow channels.

5. The automatic perfusion apparatus for cryopreservation of biological tissues according to claim 4, wherein the sample carrying plate and the flow channel plate in the perfusion module may be fabricated in different structures according to different biological samples.

6. The automatic perfusion apparatus for cryopreservation of biological tissues according to claim 5, wherein the biological sample is selected from cells, viruses, organoids, tissues, oocytes, sperm, embryos, and various physiological or pathological organ sections, such as sections of the heart, liver, lung, ovary, kidney, cartilage, blood vessels, valves, or cornea.

7. An automatic perfusion method for cryopreservation of biological tissues, comprising the following steps:
S01, selecting a corresponding sample carrying plate according to a biological sample to be perfused, and placing the biological sample in the sample carrying plate;
S02, selecting, via a display assembly, a perfusion control protocol corresponding to the biological sample, wherein the perfusion control protocol involves loading a working curve adapted to the corresponding biological sample and types of reagent solutions into a data processing system to form the perfusion control protocol;
controlling, by the data processing system on the basis of the perfusion control protocol, working states of a temperature source and peristaltic pumps, and adjusting output power of the temperature source so that real-time temperature of a sample carrying module follows a temperature-time working curve; adjusting feed rate of the peristaltic pumps so that the feed rate follows a corresponding perfusion fluid flow rate-time working curve; and adjusting aspiration by the peristaltic pumps from each reagent container so that an aspirated liquid follows a corresponding perfusion liquid composition-time working curve;
S03, aspirating, by the peristaltic pumps under the control of a perfusion protocol, liquids from the corresponding reagent containers before merging into a mixing tube; and after uniform mixing in the mixing tube, the liquids entering a perfusion module through flow channels, flowing and diffusing into sample carrying spaces or carrying channels of the sample carrying plate to perfuse the biological sample;
S04, excess mixed liquid in the sample carrying spaces or carrying channels, or liquid exuded from the biological sample flowing out through outlets of the flow channels and being collected; and
S05, removing, upon completion of the perfusion protocol, the biological sample alone or the sample carrying plate carrying the biological sample, placing in a cooling apparatus, and then placing in liquid nitrogen or a low-temperature storage device.

8. The automatic perfusion method for cryopreservation of biological tissues according to claim 7, wherein the feed rate corresponding to the perfusion fluid flow rate-time working curve is converted into operating frequencies or operating speeds of the peristaltic pumps, and the data processing module reads the operating frequency or the operating speed of each peristaltic pump and adjusts the feed rate of each peristaltic pump to realize precise control of the feed rate of the reagent solutions so that each reagent solution conforms to the perfusion fluid flow rate-time working curve.

9. The automatic perfusion method for cryopreservation of biological tissues according to claim 8, wherein the data processing module receives a signal of the real-time temperature of the sample carrying plate and determines whether the real-time temperature of the sample carrying plate corresponds to a temperature value at the current time point on the temperature-time working curve; if so, the data processing module maintains output power of a temperature-control module according to the temperature-time working curve; if not, the data processing module adjusts output power of a cooling module of the temperature-control module to compensate for temperature deviation of the sample carrying plate so that the real-time temperature of the sample carrying plate reaches the temperature value at the current time point on the temperature-time working curve; and thereafter the temperature-control module is restored to output power corresponding to the temperature-time working curve.

10. The automatic perfusion method for cryopreservation of biological tissues according to claim 8, wherein the biological sample is a biological sample to be revived from a low-temperature storage device.
